# EUROPÄISCHE PATENTANMELDUNG

(11) **EP 2 532 411 A1**
(43) Veröffentlichungstag der Anmeldung: **12.12.2012**
(21) Anmeldenummer: 11004796.6
(22) Anmeldetag: 10.06.2011
(51) Int. Cl.: B01D 53/047, B01D 61/36, B01D 69/14, B01D 71/02, B01D 71/36, C07C 45/78, C12P 7/06

(54) **Druckwechseladsorptionsverfahren**

(71) Anmelder: Süd-Chemie AG, 80333 München (DE)
(72) Erfinder: Koch, Achim, Dr., 85368 Moosburg (DE); Zavrel, Michael, Dr., 81379 München (DE); Kröhnke, Christoph, Dr., 81377 München (DE)
(74) Vertreter: HOFFMANN EITLE

(57) **Zusammenfassung**

Die vorliegende Erfindung beschreibt ein Verfahren zur Anreicherung mindestens einer Komponente aus einem gasförmigen Stoffgemisch, umfassend die Schritte
(i) Inkontaktbringen eines Stroms eines ersten gasförmigen Stoffgemisches enthaltend wenigstens eine anzureichernde Komponente mit einem Verbundmaterial bei einem ersten Druck p1, so dass die wenigstens eine anzureichernde Komponente an das Verbundmaterial adsorbiert wird und ein beladenes Verbundmaterial erhalten wird, wobei das Verbundmaterial umfasst
(a) eine poröse Matrix aus einem fluorhaltigen Polymer mit einem Anteil von Tetrafluorethylen-Monomereinheiten von wenigstens 95 mol-% bezogen auf die gesamte Anzahl der Monomereinheiten und
(b) Zeolith-Partikeln, welche in die Matrix eingebettet und von ihr umgarnt sind;

(ii) Unterbrechen des Stroms des gasförmigen Stoffgemischs und
(iii)Desorbieren der wenigstens einen anzureichernden Komponente von dem beladenen Verbundmaterial durch Vermindern des Drucks auf einen Druck p2, wobei gilt p1 - p2 ≥ 200 mbar, so dass ein zweites gasförmiges Stoffgemisch gebildet wird und Ableiten des zweiten gasförmigen Stoffgemischs von dem Verbundmaterial.

## Beschreibung

### Gebiet der Erfindung

Die Erfindung betrifft ein Verfahren zur Anreicherung mindestens einer Komponente aus einem Fluid durch Druckwechseladsorption.

### Hintergrund der Erfindung

Die Trennung von flüssigen oder gasförmigen Stoffgemischen mit Hilfe fester Materialien wird seit Jahrzehnten für eine Vielzahl von Anwendungen kommerziell bzw. industriell betrieben. Aufgrund ihrer hervorragenden Selektivitäten finden Zeolithe dabei verstärkt Anwendung.

Die Zeolithe werden in der Regel auf Träger aufgebracht. Stand der Technik ist die Verwendung von Granulaten, Formkörpern oder Membranen. Als Bindemittel eignen sich inerte Feststoffe oder Polymere, die zusammen mit den Zeolithen sorptiv wirkende Verbundmaterialien ergeben.

Ein Beispiel für ein solches Verbundmaterial wird in EP 0 773 829 beschrieben. Dabei wird ein hydrophobes Molsieb mit Porendurchmesser 5.5-6.2 Ångstrom im Verhältnis 40:1 bis 1:40 als selektives Sorptionsmedium in fibrilliertes Polytetrafluorethylen (PTFE) oder geblasene Mikrofasern (Polyamid, Polyester, Polyurethan, Polyolefin etc.) eingebettet. Die Verarbeitung erfolgt unter Zuhilfenahme eines flüssigen Gleitmittels. Die teigartige Masse wird biaxial kalandriert, wodurch sich schließlich nach dem Trocknen ein poröser Film ergibt, dessen Porosität von der Menge an Gleitmittel abgeleitet werden kann. Ähnliche Verbundmaterialien werden in den Patenten US 4153661, US 4460642 und US 5071610 beschrieben, die ebenso poröse faserförmige Membranen auf Basis von PTFE zur Einarbeitung von Sorbentien oder katalytisch wirksamen partikelförmigen Stoffen beschreiben.

PTFE ist als Matrixpolymer besonders geeignet, weil es fibrillierbar, thermisch stabil, chemisch inert und hydrophob ist, d.h. es ist zu stabilen und hochflexiblen Faservliesen verarbeitbar, im Arbeitstemperaturbereich von -250 bis +260 °C verwendbar, nimmt weder Wasser auf, noch ist es löslich; zudem ist PTFE gegenüber Säuren und Laugen weitgehend inert. PTFE ist teilkristallin und oberhalb der Phasenübergangstemperatur von 19°C fibrillierbar, d.h. durch die Anwendung von Scherkräften auf PTFE-Pulver oder die in der Dispersion enthaltenen PTFE-Kugeln lassen sich die im Material enthaltenen Kristallite zu dünnen Filamenten abspulen (dieser Effekt ist oberhalb 30°C noch besser zu beobachten; hier liegt der zweite Phasenübergang von PTFE). Diese im besten Fall nur wenige Moleküllagen dicken Filamente sind imstande, durch geeignete Verarbeitungstechnik große Mengen Füllstoff zu umgarnen, einzubetten und festzuhalten, wodurch hochgradig vernetzte, hochgefüllte PTFE-Füllstoff-Komposite erhalten werden. Darüber hinaus verhaken und verschlaufen die Polymerfasern bei der Scherung miteinander, was dem Verbundmaterial eine gewisse mechanische Stabilität verleiht. Die Verarbeitbarkeit zu Filmen und Formkörpern kann allerdings aufgrund hoher benötigter Verformungskräfte abhängig vom Füllstoff stark behindert sein, weshalb es sich durchgesetzt hat, soweit möglich Gleitmittel zu verwenden (Wasser, Alkohole, Erdöldestillate, Kohlenwasserstoffe und andere Lösungsmittel), die den Verarbeitungsprozess erleichtern, die Fibrillierung unterstützen und das vorzeitige Zerstören/Zerreißen der Fasern durch zu starke Scherung unterbinden sollen. Nach der Formgebung wird das eingearbeitete Lösungsmittel in der Regel durch Erhitzen entfernt, wodurch ein zusätzlicher definierter Grad an Porosität zurückbleibt. Durch einen optionalen Sinterprozess des PTFE-Materials bei Temperaturen um 330°C, aber unter 360°C (beginnende Zersetzung) erhält das Kompositmaterial seine endgültige Stabilität und Formgebung.

Beispiele für den Einsatzbereich solcher Verbundmaterialien sind die MembranverfahrenPervaporation oder Dampfpermeation mit sogenannten Misch-Matrix-Membranen (mixed matrix membranes, MMMs) , aber auch verschiedene Adsorptionsverfahren, wie die sogenannte Festphasenextraktion (solid phase extraction, SPE) oder Trocknungsverfahren.

Unter Festphasenextraktion ist der physikalische Trennprozess zwischen einer fluiden und einer festen Phase zu verstehen, wobei die zu isolierende bzw. zu analysierende Komponente in einem flüssigen oder gasförmigen Lösungsmittel gelöst ist. Die SPE findet unter anderem Anwendung in der analytischen oder präparativen Chromatographie (z.B. high performance liquid chromatography, HPLC, oder Gas-Chromatographie, GC).

Für Trocknungsverfahren kommen in der Regel Schüttungen aus Adsorptionsmaterialien zum Einsatz, die beim Durchströmen des Fluids durch die Schüttung Wasser adsorbieren. Bekanntestes Beispiel ist die Absolutierung von Ethanol mit Hilfe der hydrophilen Zeolithe 3A, 4A, 5A oder 13X.

Aus EP 0 773 829 ist bekannt, dass auch organische Komponenten aus einem Fluid, also aus einer Flüssigkeit oder einem Gas, adsorbiert werden können.

Um anschließend die Komponenten in angereicherter, d.h. höher konzentrierter, oder reiner Form zu gewinnen, müssen die organischen Komponenten desorbiert werden. Für die Desorption gibt es folgende Möglichkeiten:
Erstens können die adsorbierten Komponenten durch andere Komponenten verdrängt werden. Dieses Verfahren hat jedoch den Nachteil, dass nach der Desorption das Adsorptionsmittel mit den zur Verdrängung eingesetzten Komponenten beladen ist und daher weitere Schritte notwendig sind, um diese zu entfernen.

Zweitens kann die Temperatur des Adsorptionsmittels erhöht werden, bis die adsorbierten Komponenten thermisch desorbieren. Diese Möglichkeit wird in EP 0 773 829 erwähnt. Dieses Verfahren hat jedoch den Nachteil, dass es mit zunehmender Größe der Adsorbersäulen immer schwieriger wird, die Wärme über die Adsorberwände einzubringen, da das Wandflächen-zu-Volumen-Verhältnis immer niedriger und damit ungünstiger wird. Auch das Erwärmen des Adsorptionsmittels über beispielsweise heißes Spülgas ist aufgrund der geringen Wärmekapazität von Gasen mit sehr großen Volumenströmen des Spülgases verbunden. Zudem lassen sich über dieses sogenannte Temperature-Swing-Adsorptionsverfahren (TSA) in der Regel keine kurzen Taktzeiten realisieren, da das Aufwärmen und das Abkühlen sehr lange dauern können.

Die dritte Möglichkeit stellt das sogenannte Pressure-Swing-Adsorptionsverfahren (PSA) dar, bei dem die adsorbierten Komponenten durch Absenkung des Druckes desorbiert werden. Der Vorteil dieses Verfahrens ist, dass der Druck sehr schnell und gleichmäßig in der gesamten Adsorptionssäule abgesenkt und anschließend wieder angehoben werden kann. Dadurch sind sehr kurze Taktzeiten möglich. Kurze Taktzeiten ermöglichen es, die benötigte Menge an Adsorptionsmittel zu reduzieren, so dass die Adsorbersäulen deutlich kleiner dimensioniert werden können. Dies verringert nicht nur die Kosten für die Adsorptionsmittel und die Investitionskosten für die Säulen, sondern verringert auch die Betriebskosten der Adsorptionsanlagen, da kürzere Adsorberbetten auch geringere Druckverluste beim Durchströmen des gasförmigen Stoffgemischs durch die Adsorbensschüttung bedingen, die durch Pumpen oder Verdichter überwunden werden müssen.

Der Nachteil der PSA-Verfahren ist jedoch, dass das Adsorptionsmittel häufigen Druckwechseln ausgesetzt ist, was zu einer mechanischen Beanspruchung des Adsorptionsmittels führt. Typischerweise handelt es sich bei dem Adsorptionsmittel um eine Schüttung aus Feststoffpartikeln, welche mit jedem Druckwechsel aneinander reiben, so dass es zu Abrieb kommt. Bei einer Granulierung oder Verformung des Adsorptionsmittels gebildete Hohlräume können bei Druckschwankungen zum Brechen der Granulate oder Formkörper führen. Desweiteren kann es zum Bersten und Zerbrechen der Partikel kommen. Dies kann die Standzeit des Adsorptionsmittels reduzieren und aufgrund der abnehmenden mittleren Partikelgröße den Druckverlust immer weiter erhöhen.

Um diese unerwünschten Effekte so weit wie möglich zu reduzieren, werden im Wesentlichen zwei Verfahrensvarianten genutzt:
(a) Die Druckwechsel werden möglichst langsam durchgeführt, das heißt, dass die Vakuumpumpe beim Entspannen nur langsam angefahren wird, und dass beim Druckerhöhen das gasförmige Stoffgemisch nur langsam in die Adsorbersäule eingeströmt wird. Diese Zeiten für die Druckwechsel stehen demnach nicht für die Adsorption bzw. Desorption beim Zieldruck zur Verfügung. Dass die Adsorption unterhalb des Zieldrucks und die Desorption oberhalb des Zieldrucks weniger effizient ablaufen, d.h. es wird weniger Substanz pro Zeiteinheit adsorbiert bzw. desorbiert, muss durch eine Verlängerung der Taktzeit kompensiert werden. Die Zeiteffizienz des gesamten Verfahrens wird damit in unvorteilhafter Weise vermindert.
(b) Die Taktzeit wird verlängert, indem die Adsorptionsphase und/oder die Desorptionsphase verlängert wird, um die Anzahl der Druckwechsel und damit die Anzahl der Verfahrensschritte, die mit einer starken mechanischen Beanspruchung des Adsorbermaterials verbunden sind, zu reduzieren. Doch jede Verlängerung der Adsorptionsphase und/oder der Desorptionsphase erfordert größere Mengen Adsorptionsmittel, so dass die Kosteneffizienz des Verfahrens beeinträchtigt wird.

Somit führt die mechanische Beanspruchung des Adsorptionsmaterials beim Druckwechsel zu einer Minderung der Effizienz des Verfahrens.

Zusammenfassend lässt sich feststellen, dass ein optimales PSA-Verfahren ein Adsorptionsmittel verwenden sollte, welches geringe Druckverluste aufweist und kurze Taktzeiten ermöglicht, ohne dass dies die Standzeiten des Adsorptionsmittels beeinträchtigt.

### Aufgabe des erfindungsgemäßen Verfahrens

Vor dem Hintergrund des Stands der Technik bestand die Aufgabe der Erfindung darin, ein Verfahren zur Anreicherung mindestens einer Komponente aus einem Gas durch Druckwechseladsorption bereitzustellen, das insbesondere die oben genannten mit der mechanischen Beanspruchung des Adsorbermaterials verbundenen Nachteile nicht aufweist.

### Beschreibung des erfindungungsgemäßen Verfahrens

Es konnte überraschend gefunden werden, dass die gestellte Aufgabe gelöst werden kann durch ein Verfahren umfassend die folgenden Schritte:
(i) Inkontaktbringen eines Stroms eines ersten gasförmigen Stoffgemisches enthaltend wenigstens eine anzureichernde Komponente mit einem Verbundmaterial bei einem ersten Druck p1, so dass die wenigstens eine anzureichernde Komponente an das Verbundmaterial adsorbiert wird und ein beladenes Verbundmaterial erhalten wird, wobei das Verbundmaterial besteht aus
   (a) einer porösen Matrix aus einem fluorhaltigen Polymer mit einem Anteil von Tetrafluorethylen-Monomereinheiten von wenigstens 95 mol-% bezogen auf die gesamte Anzahl der Monomereinheiten;
   (b) Zeolith-Partikeln, welche in die Matrix eingebettet und von ihr umgarnt sind;
   (c) optional mindestens einem metallischen Werkstoff;
   (d) optional wenigstens einer weiteren Komponente,
      wobei
      die Menge des metallischen Werkstoffs c) 0 bis 90 Gew.-% bezogen auf die Summe des Gewichts aller Bestandteile beträgt,
      das Verhältnis des Gewichts der Komponente a) zu der Summe des Gewichts der Komponenten b) und d) 2:98 bis 25:75 beträgt, und
      das Verhältnis des Gewichts der Komponente b) zum Gewicht der Komponente d) 80:20 bis 100:0 beträgt;
(ii) Unterbrechen des Stroms des gasförmigen Stoffgemischs und
(iii)Desorbieren der wenigstens einen anzureichernden Komponente von dem beladenen Verbundmaterial durch Vermindern des Drucks auf einen Druck p2, wobei gilt p1 - p2 ≥ 200 mbar, so dass ein zweites gasförmiges Stoffgemisch gebildet wird, und Ableiten des zweiten gasförmigen Stoffgemischs von dem Verbundmaterial.

Nachfolgend werden das erfindungsgemäße Verfahren und bevorzugte Ausführungsformen detaillierter beschrieben.

### - Schritt (i)

Im ersten Schritt des Verfahrens wird bei einem Druck p1 ein erstes Gasgemisch über ein Verbundmaterial geführt, das einen Zeolithen als Adsorber enthält.

Das Verbundmaterial umfasst ein fluorhaltiges, fibrillierbares Polymer, bevorzugt PTFE, und einen Zeolithen, der zur Adsorption kleiner Moleküle geeignet ist. Zur Versteifung des Materials kann Metall in Form von Metallgitter, -gewebe, -netz, gelochten oder gestanzten Metallplatten eingearbeitet sein.

### (a) Matrix aus fluorhaltigem Polymer

Die Matrix des Verbundmaterials besteht aus fluorhaltigem Polymer, d.h. einem Homo- oder Copolymer mit einem Anteil von Tetrafluorethylen-Monomereinheiten von wenigstens 95 mol-%. Das fluorhaltige Polymer ist fibrillierbar und kann durch Fibrillierung eine poröse Matrix bilden. Das fluorhaltige Polymer ist zudem chemisch inert und in Gegenwart von Wasser oder organischen Molekülen nicht quellbar. Vorzugsweise umfasst das fluorhaltige Polymer einen Anteil von Tetrafluorethylen-Monomereinheiten von wenigstens 99 mol-%.

Als Beispiele für das fluorhaltige Polymer können Polytetrafluorethylen (PTFE), Tetrafluorethylen-Hexafluorpropylen-Copolymer, Tetrafluorethylen-Chlortrifluorethylen-Copolymer, Tetrafluorethylen-Perfluor-(2,2-dimethyl-1,3-dioxol)-Copolymer und Tetrafluorethylen-Perfluor(C₁₋₆-alkylvinylether)-Copolymer wie etwa Tetrafluorethylen-Perfluor(butenylvinylether)-Copolymer genannt werden. PTFE ist bevorzugt.

Das Polymer kann als Pulver oder Dispersion eingesetzt werden. Bevorzugt werden tensidfreie PTFE-Pulver verwendet, denn das Nichtvorhandensein von oberflächenaktiven Substanzen, die für die Haltbarkeit von PTFE-Dispersionen notwendig sind, eliminiert die unerwünschten Effekte der Reduktion der verfügbaren Zeolith-Oberfläche bzw. Erhöhung der Wasseradsorption durch ebensolche Tenside.

Diese Polymere sind gemäß den in EP 0 773 829 B1 (und darin zitierten Dokumenten des Stands der Technik) beschriebenen Verfahren fibrillierbar, so dass eine poröse und faserige Matrix gebildet wird.

### (b) Zeolith-Partikel

Für das erfindungsgemäße Verbundmaterial sind insbesondere Sorbentien geeignet, die Moleküle aus gasförmigen Mischungen selektiv sorbieren und unter geeigneten Bedingungen wieder desorbieren können. Geeignet hierfür sind insbesondere Zeolithe.

Für das Verbundmaterial sind weiterhin jene Sorbentien interessant, die geeignet sind, organische polare Moleküle aus wasserenthaltenden Fluiden zu sorbieren und unter geeigneten Bedingungen wieder zu desorbieren, um sie anzureichern oder aufzureinigen. Geeignet hierfür sind insbesondere hydrophobe Zeolithe, d.h. Zeolithe mit einem SiO₂:Al₂O₃-Verhältnis von 100:1 oder mehr, bevorzugt 200:1 oder mehr, weiter bevorzugt 500:1 oder mehr. Diese Zeolithe sind für die Adsorption von organischen Molekülen wie Alkoholen (z.B. Ethanol, Butanol), Ethern, Ketonen (z.B. Aceton), Aldehyden (z.B. Acetaldehyd), Estern (z.B. Ethylacetat), Carbonsäuren (z.B. Essigsäure) und Carbonsäureestern etc. generell gut geeignet. Die Bestimmung des SiO₂:Al₂O₃-Verhältnisses erfolgt durch Röntgenfluoreszenzspektroskopie (XRF) einer bei 100 °C eine Stunde lang getrockneten Probe, die anschließend mit einem Bindemittel zu einer Tablette gepresst wird, durch Bestimmung des molaren Verhältnisses von Si:Al, das zum molaren Verhältnis SiO₂ : Al₂O₃ umgerechnet wird.

Um besonders gute Adsorptionseigenschaften aufzuweisen, d.h. eine große Anzahl Moleküle pro Gewichtseinheit Zeolith adsorbieren zu können, sollten die Zeolithe eine große, durch die BET-Methode bestimmte Oberfläche pro Gewichtseinheit aufweisen. Für die vorliegende Erfindung geeignete Zeolithe weisen eine Oberfläche lt. BET-Methode von 150 m²/g oder größer, bevorzugt 200 m²/g oder größer, noch bevorzugter von 300 m²/g oder größer auf.

Die Oberfläche wird mit einem vollautomatischen Stickstoffporosimeter der Firma Micromeritics Typ ASAP 2010 unter Verwendung von Stickstoff als adsorbiertem Gas gemäß dem folgenden Verfahren nach DIN 66131 (Juli 1993) bestimmt. Die Probe wird im Hochvakuum auf die Temperatur von flüssigem Stickstoff abgekühlt. Anschließend wird kontinuierlich Stickstoff in die Probenkammern dosiert. Durch die Erfassung der adsorbierten Gasmenge als Funktion des Druckes wird bei konstanter Temperatur eine Adsorptionsisotherme ermittelt. In einem Druckausgleich wird das Analysengas schrittweise entfernt und eine Desorptionsisotherme aufgenommen. Zur Ermittlung der spezifischen Oberfläche und der Porosität nach der BET-Theorie werden die Daten gemäß DIN 66131 (Juli 1993) ausgewertet.

Unter diesen Gesichtspunkten bevorzugt sind Zeolithe der Typen Silicalit, β-Zeolith, Mordenit, Y-Zeolith, MFI-Zeolith, Ferrierit (FER-Zeolith), dealuminierter, ultrastabiler Zeolith Y (USY-Zeolith) und Erionit (ERI-Zeolith). Das erfindungsgemäße Verfahren lässt auch Mischungen dieser Zeolithe zu.

Es werden vorzugsweise Zeolith-Partikel der Teilchengröße (d₅₀) von 0,5 bis 100 µm, weiter bevorzugt von 1 bis 50 µm und besonders bevorzugt von 5 bis 25 µm eingesetzt. Grundsätzlich nimmt mit abnehmender Teilchengröße die spezifische Oberfläche, d.h. die Oberfläche pro Masseneinheit zu. Eine große spezifische Oberfläche führt in der Regel zu einer hohen und damit vorteilhaften Adsorptionsgeschwindigkeit. Da die Handhabung und Verarbeitung eines Pulvers jedoch mit abnehmender Teilchengröße zunehmend schwierig und aufwendig wird, wäre es nicht vorteilhaft, sehr geringe Teilchengrößen zu wählen, wenngleich dies prinzipiell möglich ist.

Es kann ein einzelner Zeolith-Typ oder eine Mischung aus mehreren Zeolith-Typen eingesetzt werden. Der einzelne Zeolith-Typ oder die Zeolith-Typen können in einer einheitlichen Teilchengröße oder in mehreren Teilchengrößen eingesetzt werden.

### (c) Metallischer Werkstoff

Das Verbundmaterial kann einen metallischen Werkstoff enthalten.

Für das erfindungsgemäße Verbundmaterial eignen sich metallische Werkstoffe, d.h. reine Metalle und Legierungen. Insbesondere eignen sich metallische Werkstoffe, die in Gegenwart von Wasser und organischen Molekülen chemisch inert sind, d.h. nicht oder nur eingeschränkt mit Wasser und/oder organischen Verbindungen reagieren. Eingeschränktes Reagieren mit Wasser und/oder organischen Verbindungen bedeutet beispielsweise, dass eine Passivierung der Oberfläche des metallischen Werkstoffs auftritt, aber keine chemische Reaktion, die schließlich zum vollständigen Abbau des metallischen Werkstoffs führt.

Unter diesen Gesichtspunkten bevorzugt sind korrosionsfreie Metalle, besonders bevorzugt Edelstähle, die in der Lebensmittel- und chemischen Industrie verwendet werden, z.B. X2CrNi1911 (Werkstoffnummer = WNr. 1.4306), X12CrNi177 (WNr. 1.4310), oder X5CrNi1810 (WNr. 1.4301).

Die Form, in der der metallische Werkstoff in dem Verbundmaterial vorliegt, ist nicht beschränkt. Beispielsweise kann der metallische Werkstoff in flächiger Form, d.h. beispielsweise in Form von Metallgittern, -geweben, -netzen, sowie gelochten oder gestanzten Metallplatten oder -blechen, oder in Teilchenform, d.h. beispielsweise in Form von Pulvern oder Spänen, in dem Verbundmaterial vorliegen. Durch die als Beispiele genannten Strukturen wird sichergestellt, dass eine gute Verbindung zwischen Metall und Verbundmaterial erzielt wird. Der metallische Werkstoff kann in mehreren Formen in dem Verbundmaterial vorliegen, d.h. sowohl in Teilchenform als auch in flächiger Form.

Bei der Verwendung des metallischen Werkstoffs in flächiger Form wird eine Maschenweite bzw. Lochöffnung von 0,5-5 mm, insbesondere von 1-2 mm bevorzugt. Die Anzahl und Verteilung von Löchern pro Flächeneinheit ist nicht sonderlich beschränkt und wird durch Überlegungen des Fachmanns hinsichtlich der gewünschten Durchlässigkeit und der Stabilität bestimmt. Ebenso ist die Dicke des metallischen Werkstoffs in der eingesetzten flächigen Form nicht sonderlich beschränkt, sofern die gewünschte Dimensionsstabilität erreicht wird. Zu diesem Zweck beträgt die Dicke des metallischen Werkstoffs gängigerweise 0,1-1 mm, bevorzugt 0,2-0,5 mm, besonders bevorzugt 0,25 mm.

In dem erfindungsgemäßen Verbundmaterial beträgt die Menge des optionalen metallischen Werkstoffs (c) 0 bis 90 Gew.-% bezogen auf die Summe aller Bestandteile des Verbundmaterials, d.h. der metallische Werkstoff ist eine optionale Komponente. Wenn der metallische Werkstoff vorhanden ist, beträgt die Menge des metallischen Werkstoffs (c) mehr als 0 Gew.-%, aber nicht mehr als 90 Gew.-%, bezogen auf die Summe aller Bestandteile des Verbundmaterials. Vorzugsweise beträgt die Menge des metallischen Werkstoffs c) 5 bis 80 Gew.-%, weiter bevorzugt 10 bis 70 Gew.-%, bezogen auf die Summe aller Bestandteile des Verbundmaterials.

### (d) Weitere Komponente

In dem erfindungsgemäßen Verbundmaterial können optional eine oder mehrere Komponenten vorhanden sein, die beispielsweise aus Hilfsstoffen, Tensiden, Gleitmitteln, Fällungskieselsäure, Silica, Aktivkohle, Pigmenten, Glaskugeln oder -fasern, Kunststofffasern, Fasern natürlichen Ursprungs, Tonmineralen wie etwa Bentonit ausgewählt sein können.

Das fluorhaltige Polymer (a) liegt in einem Verhältnis zum Gesamtgewicht der Zeolith-Partikel (b) und der optional vorhandenen weiteren Komponente (d) von 2:98 bis 30:70, vorzugsweise von 4:96 bis 20:80, weiter bevorzugt von 5:95 bis 15:85.

Das Verhältnis des Gewichts der Zeolith-Partikel (b) zum Gewicht der Komponente (d) beträgt 80:20 bis 100:0, d.h. die Komponente (d) ist optional. Vorzugsweise beträgt das Verhältnis des Gewichts der Zeolith-Partikel (b) zum Gewicht der Komponente (d) 90:10 bis 100:0, weiter bevorzugt 95:5 bis 100:0.

In einer bevorzugten Ausführungsform liegt das Verhältnis des Gewichts des fluorhaltigen Polymers (a) zum Gesamtgewicht der Zeolith-Partikel (b) und der optional vorhandenen weiteren Komponente (d) in einem Bereich von 4:96 bis 20:80, weiter bevorzugt 5:95 bis 15:85, wobei das Verhältnis des Gewichts der Zeolith-Partikel (b) zum Gewicht der Komponente (d) 90:10 bis 100:0 beträgt.

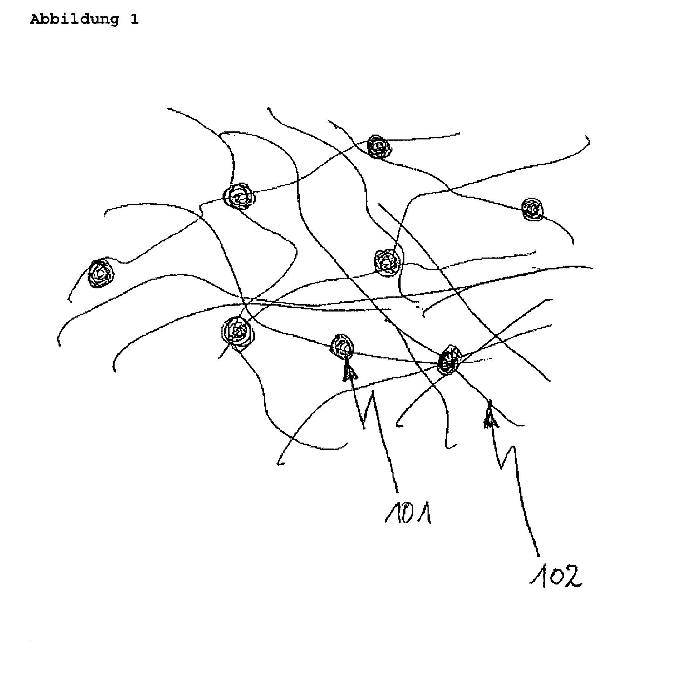

Das Verbundmaterial wird durch Vermischen der Komponenten (a) und (b) und des optionalen metallischen Werkstoffs (c), sofern der metallische Werkstoff (c) in einer geeigneten kleinteiligen Form, also beispielsweise in Pulverform, verwendet wird, in den oben genannten Mengen und anschließendem Kneten hergestellt, wobei sich unter Scherung die Fibrillierung des Polymers und Einarbeitung des Zeolithen in die poröse Polymermatrix einstellt [Abbildung 1]. Das Kneten wird bei Raumtemperatur oder vorzugsweise bei einer erhöhten Temperatur wie etwa 30 °C oder mehr, 50 °C oder mehr oder 70 °C oder mehr durchgeführt, da bei einer Temperatur in diesen Bereichen in der Regel eine bessere Verarbeitbarkeit und insbesondere eine bessere Fibrillierung des fluorhaltigen Polymers möglich ist. Die Temperaturobergrenze wird in erster Linie durch thermische Stabilität der in der Mischung enthaltenen Komponenten bestimmt. Unter diesem Gesichtspunkt ist in der Regel eine Verarbeitung bei einer Temperatur von nicht mehr als 200 °C bevorzugt, weiter bevorzugt von nicht mehr als 150 °C.

Um eine gute Mischbarkeit der Komponenten des Verbundmaterials zu erreichen, werden Polymer (a) und Zeolith (b) bevorzugt in Pulverform eingesetzt. Das Polymer (a) kann beispielsweise auch in Form einer handelsüblichen Dispersion in Wasser eingesetzt werden. Solche handelsüblichen Dispersionen können Hilfsstoffe wie etwa Stabilisatoren, Tenside oder andere, die Oberflächenspannung verändernde Komponenten und/oder andere Hilfsstoffe enthalten.

Um den Misch- und Scherprozess zu erleichtern, kann als Gleitmittel Wasser oder Alkohol zugesetzt werden. Um später auf einen energie- und kostenintensiven Trockenschritt weitgehend verzichten zu können, wird aber bevorzugt mit möglichst wenig Flüssigkeit gearbeitet, d.h. außer der über die PTFE-Dispersion (maximal 40% der Dispersion) zugeführten Flüssigkeitsmenge wird kein Gleitmittel zugesetzt.

Nach dem Knetschritt wird das teig- bis vliesartige Produkt zwischen beheizten Walzen (Temperatur 60-150°C) in mehreren Schritten biaxial zunächst zu einer Matte, dann zu einem Film ausgewalzt, wobei die Fibrillierung optimiert und beispielweise eine homogene End-Schichtdicke von 0,3 bis 1 mm, bevorzugt 0,4-0,6 mm eingestellt wird. Geeignet für diesen Schritt ist ein beheizbares Kalander- oder Rollwalzsystem aus mindestens 2 Rollen, bevorzugt 4 Rollen oder mehr.

Ein geeignetes Verfahren zur Herstellung eines Verbundmaterials aus einem Polymer (a) und einem Zeolith (b) wird auch in EP 0 773 829 B1 und darin zitierten Dokumenten beschrieben.

Wenn ein metallischer Werkstoff in einer flächigen Form eingebracht werden soll, wird das so erhaltene Material in einem oder mehreren Schritten zwischen druckbelasteten Walzen in einem Laminator oder Kalander so mit dem metallischen Werkstoff in flächiger Form, z.B. Edelstahlgeflecht, verpresst, dass ein Verbund aus mindestens einer Lage des Materials und dem metallischen Werkstoff gebildet wird. Bevorzugt wird eine Lage des metallisches Werkstoffs zwischen zwei Lagen des Materials eingeschlossen. Bevorzugt durchdringen sich beide Schichten des Materials durch die Öffnungen in dem flächigen metallischen Werkstoff hindurch, wodurch die Stabilität des Verbundes optimiert wird. Der Schritt des Verbindens des metallischen Werkstoffs und des Materials kann bei Raumtemperatur, vorteilhaft aber bei 70-250°C geschehen, um Restfeuchte zu vertreiben, die beispielweise aufgrund der Verwendung von Wasser als Gleitmittel beim Mischen und/oder Kneten von fluorhaltigem Polymer (a) und Zeolith-Partikeln (b) wie oben beschrieben in dem Material vorliegen kann. Optional schließt sich ein Trocknungsschritt an.

Optional wird ein oder mehrere Heizelement(e) in das Material so eingebracht, dass die Wärmeenergie vom Heizelement an das metallische Material gut übertragen werden kann. Der metallische Werkstoff kann ggf. selbst die Funktion des Heizelementes übernehmen, z.B. durch Beheizung mittels magnetischer Induktion, elektrische Widerstandsheizung oder Wärmeaustausch. Durch das Heizelement kann die Adsorptions-und Desorptionstemperatur im Sinne der Prozessausbeute optimiert werden. Es dient außerdem zur Erleichterung der ggf. notwendigen Regeneration des Materials.

Das Verbundmaterial kann in beliebiger Form verwendet werden. Beispielsweise kann das Verbundmaterial in Form eines gefalteten oder spiralförmig gewickelten Films oder in Form einer Schüttung einer Vielzahl von Formkörpern oder Teilchen angeordnet sein, um mit dem Strom des ersten gasförmigen Stoffgemischs in Kontakt gebracht zu werden. Es können auch verschiedene Formen in Kombination verwendet werden. Die Anordnung des Verbundmaterials in dem Strom des ersten gasförmigen Stoffgemischs wird nachfolgend auch als "Packung" bezeichnet.

Das erste gasförmige Stoffgemisch enthält wenigstens eine anzureichendernde Komponente. Diese Komponente kann organisch oder nicht-organisch sein und gegenüber weiteren Komponenten anzureichern zu sein, die organisch oder nicht-organisch sind.

Beispielsweise handelt es sich bei der wenigstens einen anzureichernden Komponente um eine organische Substanz, beispielsweise aus einer der Substanzklassen Alkohole (z.B. Ethanol, Butanol), Ether (z.B. Methyl-tert-butylether oder Tetrahydrofuran) Ketone (z.B. Aceton), Aldehyde (z.B. Acetaldehyd), Ester (z.B. Ethylacetat) und Carbonsäuren (insbesondere C₁₋₄-Carbonsäuren wie z.B. Essigsäure oder Propionsäure).

Als nicht-organische Komponente kann das gasförmigen Stoffgemisch beispielsweise Wasser enthalten. Als nicht-organische Komponenten können auch Schwefelwasserstoff, Ammoniak, Wasserstoff, Kohlendioxid, Sauerstoff oder Stickstoff in dem gasförmigen Stoffgemisch vorliegen.

Bevorzugt handelt es sich bei dem ersten gasförmigen Stoffgemisch um ein Stoffgemisch, das durch Gas-Stripping einer wässrigen Lösung mit flüchtigen organischen Verbindungen der oben genannten Substanzklassen erhalten wird. Besonders bevorzugt handelt es sich bei der wässrigen Lösung um eine Fermentationslösung, in der mindestens eine der oben genannten organischen Substanzen fermentativ oder enzymatisch hergestellt, ganz besonders bevorzugt um eine Fermentationslösung, die durch ethanolische Gärung mittels Hefen oder Bakterien oder durch eine sogenannte ABE-Fermentation mittels Bakterien erhalten wird. Bei einer sogenannten ABE-Fermentation werden mittels Bakterien Aceton, Butanol und Ethanol (ABE) produziert. Dieses Gas-Stripping wird besonders bevorzugt in-situ durchgeführt, wobei in-situ bedeutet, dass das Gas-Stripping während der Fermentation erfolgt. Das Gas-Stripping kann aber auch nach abgeschlossener Fermentation erfolgen. Das Gas-Stripping kann in einer mit dem Fermenter verbundenen externen Gas-Stripping-Vorrichtung erfolgen.

Die Adsorption erfolgt bei einem höheren Druck (p1) als die Desorption. Der Druck bei der Adsorption liegt beispielsweise im Bereich von 0,4 bis 20 bar, bevorzugt im Bereich von 0,6 bis 10 bar, besonders bevorzugt im Bereich von 0,8 bis 5 bar und ganz besonders bevorzugt im Bereich von 1 bis 2,5 bar.

Die Temperatur des Gasstroms beim Inkontaktbringen mit dem Verbundmaterial liegt zwischen 0 und 250 °C, bevorzugt zwischen 10 und 200 °C, besonders bevorzugt zwischen 20 und 150 °C und ganz besonders bevorzugt zwischen 30 und 100 °C.

Vorzugsweise wird die Strömungsgeschwindigkeit des ersten gasförmigen Stoffgemisches so eingestellt, dass ein Volumenstrom beim Inkontaktbringen des ersten gasförmigen Stoffgemisches mit dem Verbundmaterial im Schritt (i) erreicht wird, der einer Leerrohrgeschwindigkeit von 0,5 m/s oder mehr entspricht. Weiter bevorzugt wird die Strömungsgeschwindigkeit des ersten gasförmigen Stoffgemisches so eingestellt, dass ein Volumenstrom beim Inkontaktbringen des ersten gasförmigen Stoffgemisches mit dem Verbundmaterial im Schritt (i) erreicht wird, der einer Leerrohrgeschwindigkeit von 0,75 m/s oder mehr, besonders bevorzugt 1,0 m/s oder mehr entspricht.

Der Strom des ersten gasförmigen Stoffgemischs wird über die Packung des Verbundmaterials geführt, bis eine gewünschte Beladung erfolgt ist. Diese gewünschte Beladung muss nicht speziell bestimmt werden, sondern beispielsweise kann der Strom des ersten gasförmigen Stoffgemischs für eine bestimmte geeignet erscheinende Zeitspanne über die Packung des Verbundmaterial geführt werden.

### - Schritt (ii)

Wenn die gewünschte Beladung des Verbundmaterials erreicht ist, wird der Strom des ersten gasförmigen Stoffgemischs unterbrochen.

### - Schritt (iii)

Durch diesen Schritt wird die wenigstens eine anzureichernde Komponente von dem Verbundmaterial desorbiert und das Verbundmaterial so für eine erneute Durchführung des Schritts (i) vorbereitet. Durch die Desorption der wenigstens einen anzureichernden Komponente von dem Verbundmaterial wird mit der Atmosphäre, die das Verbundmaterial bei der Desorption umgibt, ein zweites gasförmiges Stoffgemisch gebildet.

Die Desorption der wenigstens einen anzureichernden Komponente erfolgt bei einem niedrigeren Druck (p2) als die Adsorption im Schritt (i). Die Druckdifferenz (p1-p2) zwischen dem Adsorptions- und dem Desorptionsschritt beträgt mindestens 200 mbar, bevorzugt mindestens 500 mbar, und besonders bevorzugt mindestens 800 mbar.

In einer bevorzugten Ausführungsform des erfindungsgemäßen Verfahrens wird bei der Desorption ein Spülgas verwendet, d.h. die wenigstens eine anzureichernde Komponente, die von dem Verbundmaterial desorbiert wird, wird durch ein über das Verbundmaterial geführtes Gas (das Spülgas) aus der Packung des Verbundmaterials gespült. Bevorzugte Spülgase sind inerte Gase, besonders bevorzugt sind die Spülgase Luft, Kohlenstoffdioxid, Stickstoff, Edelgase oder Mischungen daraus. In einer weiteren Ausführungsform des erfindungsgemäßen Verfahrens enthält das Spülgas Wasser. Besonders bevorzugt liegt die Temperatur des Spülgases oberhalb der Temperatur des Adsorptionsmittels.

Bevorzugt ist die Strömungsrichtung des zweiten gasförmigen Stoffgemischs umgekehrt zur Strömungsrichtung des ersten gasförmigen Stoffgemischs im Schritt (i).

In einer weiteren erfindungsgemäßen Ausführungsform des Verfahrens wird eine Mischform aus PSA und TSA angewendet, d.h. dass die Desorption nicht alleine durch Druckabsenkung, sondern durch unterstützendes Einbringen thermischer Energie erfolgt. Dieses unterstützende Einbringen thermischer Energie kann durch Einbringen heißen Spülgases oder durch Beheizung über die Säulenwand, über Heizwendeln im Inneren der Adsorbersäule oder der Packung des Verbundmaterials oder durch Kombinationen aller drei Optionen erfolgen. Wenn ein metallischer Werkstoff (c) in einer flächigen Form im Verbundmaterial vorliegt, kann das Einbringen thermischer Energie auch durch Beheizung des metallischen Werkstoffs erfolgen, beispielsweise durch magnetische Induktion, elektrische Widerstandheizung oder durch Wärmeaustausch. Wenn ein metallischer Werkstoff (c) in einer Teilchenform im Verbundmaterial vorliegt, kann das Einbringen thermischer Energie auch durch Beheizung des metallischen Werkstoffs erfolgen, beispielsweise durch magnetische Induktion oder durch Wärmeaustausch.

Die Anzahl der Druckwechsel zwischen p1 und p2 beträgt vorzugsweise mindestens 1 pro Stunde, weiter bevorzugt mindestens 2 pro Stunde und besonders bevorzugt mindestens 3 pro Stunde.

Die mittlere Geschwindigkeit ,mit der der Druckwechsel zwischen p1 und p2 durchgeführt wird, beträgt vorzugsweise 40 mbar/min oder mehr, weiter bevorzugt 100 mbar/min oder mehr, besonders bevorzugt 200 mbar/min oder mehr.

### Kurze Beschreibung der Abbildung

Abbildung 1 skizziert das PTFE-Zeolith-Verbundmaterial, das beim erfindungsgemäßen Verfahren eingesetzt wird (101 = Zeolithpartikel, 102 = netzartige PTFE-Fibrillen).

### Beispiele

Das erfindungsgemäße Verfahren wird anhand folgender, nicht einschränkender, Beispiele erläutert:

### Beispiel 1: Herstellung eines PTFE-Zeolith-Verbundmaterials

PTFE-Dispersion TE3893-N (ca. 60% PTFE Gehalt, DuPont) mit einem PTFE-Massenanteil von 10% zusammen mit einem Zeolith (ZSM-5, H-Form; SiO₂/Al₂O₃ > 800; Hersteller: Süd-Chemie AG, Deutschland), wird durch Vermischen der einzelnen Materialien und anschließendem einstündigem Kneten in einem Werner&Pfleiderer LUK 075 Laborkneter bei 80-130 °C hergestellt, wobei sich unter Scherung die Fibrillierung des PTFE und Einarbeitung des Zeolithen einstellt.

Nach dem Knetschritt wird das vliesartige Produkt zwischen beheizten Walzen (Temperatur 70 °C) in mehreren Schritten biaxial zu einem Film ausgewalzt, wobei die Fibrillierung optimiert und eine homogene Schichtdicke von 0.5 mm eingestellt wird. Geeignet für diesen Schritt ist ein Kalandersystem der Fa. Fetzel.

### Beispiel 2: Adsorption von Ethanol aus einem Gasstrom

500 mL einer 5% (w/v) -Ethanol-Wasser-Lösung wurden für 24 Stunden mit einem Volumenstrom von 1 L/min gestrippt. Es wurden eine Membranpumpe (KNF, Deutschland), ein Volumenstromregler (Swagelok, Deutschland) und eine Gaswaschflasche (VWR, Deutschland) verwendet. Der Gasstrom wurde durch eine Glassäule (VWR, Deutschland) geleitet, welche mit dem Verbundmaterial aus Beispiel 1 gepackt war. Der Gasstrom wurde im Rahmen einer Kreislaufführung in die Gaswaschflasche rückgeführt, so dass das System geschlossen war. Die Glassäule wurde über eine Heizmanschette (Mohr & Co GmbH, Deutschland) auf 40 °C erwärmt. Das Gas-Stripping in der Gaswaschflasche erfolgte bei 30 °C. Nach Versuchsende wurde die Ethanol-Konzentration über Gas-Chromatografie (Trace GC, ThermoFischer, Deutschland) in der Lösung bestimmt. Zudem wurde die Gewichtsveränderung des Zeolithen und der Lösung bestimmt. Über eine Massenbilanz wurden dann die Beladungen des Zeolithen mit Wasser und Ethanol und daraus der Wasseranteil und der Anteil der flüchtigen organischen Verbindung Ethanol berechnet.

### Beispiel 3: Ad- und Desorption von Ethanol durch Druckwechsel

500 mL einer 5% (w/v) -Ethanol-Wasser-Lösung wurden für 24 Stunden mit einem Volumenstrom von 1 L/min gestrippt. Es wurden eine Membranpumpe (KNF, Deutschland), ein Volumenstromregler (Swagelok, Deutschland) und eine Gaswaschflasche (VWR, Deutschland) verwendet. Der Gasstrom wurde durch eine Glassäule (VWR, Deutschland) geleitet, welche mit 101,7 g Verbundmaterial aus Beispiel 1 gepackt war. Der Gasstrom wurde im Rahmen einer Kreislaufführung in die Gaswaschflasche rückgeführt, so dass das System geschlossen war. Die Glassäule wurde über eine Heizmanschette (Mohr & Co GmbH, Deutschland) auf 40 °C erwärmt. Das Gas-Stripping in der Gaswaschflasche erfolgte bei 30 °C. Nach der Adsorption wurde bei 50 mbar mittels einer Vakuumpumpe (Typ CVC3000; VacuuBrand, Deutschland) desorbiert. Das Desorbat wurde in einer Kühlfalle, die mit flüssigem Stickstoff gekühlt wurde, kondensiert.

### Beispiel 4: Druckverlust

Die beiden Materialien aus den Beispielen 2 und 3 wurden hinsichtlich Druckverlust miteinander verglichen. Dazu wurden Glassäulen mit 30 mm Durchmesser mit jeweils beiden Materialien gepackt, wobei die Schütthöhe in beiden Fällen 290 mm betrug. Anschließend wurde ein Gasstrom durch die Säule geleitet, der einer Gasleerrohrgeschwindigkeit von 1 m/s entsprach. Vor der Säule wurde der Druck über einen Drucksensor gemessen, hinter der Säule herrschte atmosphärischer Druck. Die Druckdifferenz entspricht dem Druckverlust der Packung.

Bei dem PTFE-Zeolith-Material betrug der auf einen Meter hochgerechnete Druckverlust 7,5 mbar, bei den Zeolith-Granulaten 153,5 mbar. Der Druckverlust des PTFE-Zeolith-Materials betrug also weniger als 5% der Granulat-Schüttung. Auch wenn auf die gleiche Masse an Adsorptionsmittel normiert wurde, betrug der Druckverlust des PTFE-Zeolith-Materials weniger als 7,5% der Granulat-Schüttung.

## Patentansprüche

1. Verfahren zur Anreicherung mindestens einer Komponente aus einem gasförmigen Stoffgemisch, umfassend die Schritte
(i) Inkontaktbringen eines Stroms eines ersten gasförmigen Stoffgemisches enthaltend wenigstens eine anzureichernde Komponente mit einem Verbundmaterial bei einem ersten Druck p1, so dass die wenigstens eine anzureichernde Komponente an das Verbundmaterial adsorbiert wird und ein beladenes Verbundmaterial erhalten wird, wobei das Verbundmaterial besteht aus
(a) einer porösen Matrix aus einem fluorhaltigen Polymer mit einem Anteil von Tetrafluorethylen-Monomereinheiten von wenigstens 95 mol-% bezogen auf die gesamte Anzahl der Monomereinheiten;
(b) Zeolith-Partikeln, welche in die Matrix eingebettet und von ihr umgarnt sind;
(c) optional mindestens einem metallischen Werkstoff;
(d) optional wenigstens einer weiteren Komponente, wobei
die Menge des metallischen Werkstoffs c) 0 bis 90 Gew.-% bezogen auf die Summe des Gewichts aller Bestandteile beträgt,
das Verhältnis des Gewichts der Komponente a) zu der Summe des Gewichts der Komponenten b) und d) 2:98 bis 25:75 beträgt, und das Verhältnis des Gewichts der Komponente b) zum Gewicht der Komponente d) 80:20 bis 100:0 beträgt;
(ii) Unterbrechen des Stroms des gasförmigen Stoffgemischs und
(iii)Desorbieren der wenigstens einen anzureichernden Komponente von dem beladenen Verbundmaterial durch Vermindern des Drucks auf einen Druck p2, wobei gilt p1 - p2 ≥ 200 mbar, so dass ein zweites gasförmiges Stoffgemisch gebildet wird, und Ableiten des zweiten gasförmigen Stoffgemischs von dem Verbundmaterial.

2. Verfahren gemäß Anspruch 1, wobei die Konzentration der wenigstens einen anzureichernden Komponente in dem in Schritt (iii) gebildeten zweiten gasförmigen Stoffgemisch höher ist als in dem ersten gasförmigen Stoffgemisch.

3. Verfahren gemäß Anspruch 1 oder Anspruch 2, wobei der Zeolith ein hydrophober Zeolith ist.

4. Verfahren gemäß einem der Ansprüche 1 bis 3, wobei die mindestens eine anzureichernde Komponente in dem ersten gasförmigen Stoffgemisch eine organische Komponente ist.

5. Verfahren gemäß einem der Ansprüche 1 bis 3, wobei das gasförmige Stoffgemisch Wasser und mindestens eine organische Komponente ausgewählt aus der Gruppe bestehend aus Alkoholen, Ethern, Ketonen, Carbonsäuren, Carbonsäureestern und Aldehyden als die anzureichernde Komponente enthält.

6. Verfahren gemäß einem der Ansprüche 1 bis 5, wobei das Verhältnis des Gewichts der Komponente (a) zu der Summe des Gewichts der Komponenten (b) und (d) 4:96 bis 20:80 beträgt.

7. Verfahren gemäß einem der Ansprüche 1 bis 5, wobei das Verhältnis des Gewichts der Komponente (a) zu der Summe des Gewichts der Komponenten (b) und (d) 5:95 bis 15:85 beträgt.

8. Verfahren gemäß einem der Ansprüche 1 bis 7, wobei die Menge des metallischen Werkstoffs c) 1 bis 90 Gew.-% bezogen auf die Summe des Gewichts aller Bestandteile beträgt.

9. Verfahren gemäß einem der Ansprüche 1 bis 7, wobei die Menge des metallischen Werkstoffs c) 5 bis 80 Gew.-% bezogen auf die Summe des Gewichts aller Bestandteile beträgt.

10. Verfahren gemäß einem der Ansprüche 1 bis 7, wobei die Menge des metallischen Werkstoffs c) 10 bis 70 Gew.-% bezogen auf die Summe des Gewichts aller Bestandteile beträgt.

11. Verfahren gemäß einem der Ansprüche 3 bis 10, wobei der Zeolith aus der Gruppe bestehend aus Silicalit, β-Zeolith, Mordenit, Y-Zeolith, MFI-Zeolith, Ferrierit (FER-Zeolith), dealuminiertem, ultrastabilem Zeolith Y (USY-Zeolith) und Erionit (ERI-Zeolith) und deren Mischungen ausgewählt ist.

12. Verfahren gemäß einem der Ansprüche 1 bis 11, wobei p1 - p2 ≥ 500 mbar beträgt.

13. Verfahren gemäß einem der Ansprüche 1 bis 12, wobei der Wechsel zwischen dem Druck p1 und dem Druck p2 mit einer mittleren Geschwindigkeit von 40 mbar/min oder mehr erfolgt.

14. Verfahren gemäß einem der Ansprüche 1 bis 12, wobei der Wechsel zwischen dem Druck p1 und dem Druck p2 mit einer mittleren Geschwindigkeit von 100 mbar/min oder mehr erfolgt.

15. Verfahren gemäß einem der Ansprüche 1 bis 14, wobei die Strömungsrichtung des zweiten gasförmigen Stoffgemischs im Schritt (iii) entgegengesetzt der Strömungsrichtung des ersten gasförmigen Stoffgemischs im Schritt (i) ist.

16. Verfahren gemäß einem der Ansprüche 1 bis 15, wobei das Inkontaktbringen im Schritt (i) bei einem Volumenstrom erfolgt, der einer Leerrohrgeschwindigkeit von 0,5 m/s oder mehr entspricht.

17. Verfahren gemäß einem der Ansprüche 1 bis 15, wobei das Inkontaktbringen im Schritt (i) bei einem Volumenstrom erfolgt, der einer Leerrohrgeschwindigkeit von 0,75 m/s oder mehr entspricht.

18. Verfahren gemäß einem der Ansprüche 1 bis 17, wobei die Anzahl der Druckwechsel zwischen p1 und p2 mindestens 1 pro Stunde beträgt.

19. Verfahren gemäß einem der Ansprüche 1 bis 17, wobei die Anzahl der Druckwechsel zwischen p1 und p2 mindestens 2 pro Stunde beträgt.
